# EUROPEAN PATENT SPECIFICATION

(11) **EP 3 589 321 B1**
(45) Date of publication and mention of the grant of the patent: **05.10.2022**
(21) Application number: 18761791.5
(22) Date of filing: 02.03.2018
(51) Int. Cl.: A61K 31/519, A61K 31/4184, A61K 31/437, A61K 31/517, A61K 31/52, A61K 31/522, A61K 31/5517, A61K 31/4985, A61K 39/395, A61K 31/496, A61K 31/404, A61P 25/00, A61P 25/28, A61K 45/06, C12Q 1/48, G01N 33/68

(54) **METHOD AND COMPOUND FOR MODIFYING CIRCADIAN CLOCK**
VERFAHREN UND VERBINDUNG ZUR ÄNDERUNG DES BIORHYTHMUS
PROCÉDÉ ET COMPOSÉ POUR MODIFIER L'HORLOGE CIRCADIENNE

(30) Priority: 03.03.2017 WO PCT/CN2017/075570
(43) Date of publication of application: 08.01.2020
(73) Proprietor: Gritscience Biopharmaceuticals Co., Ltd., Beijing 102206 (CN)
(72) Inventor: YU, Tenghui, Beijing 100871 (CN); RAO, Yi, Beijing 100871 (CN)
(74) Representative: Protector IP AS
(86) International application number: PCT/CN2018/077909
(87) International publication number: WO 2018/157863

(56) References cited:
- WO-A1-2008/129069
- WO-A1-2015/026634
- WO-A2-2010/011331
- WO-A2-2010/121225
- Keith Petrie ET AL: "Effect of melatonin on jet lag after long haul flights", , 18 March 1989 (1989-03-18), pages 705-707, XP055664614, Retrieved from the Internet: URL:https://www.ncbi.nlm.nih.gov/pmc/artic les/PMC1835985/pdf/bmj00223-0021.pdf [retrieved on 2020-02-03]
- LING-YAN SU ET AL: "Melatonin attenuates MPTP-induced neurotoxicity via preventing CDK5-mediated autophagy and SNCA/[alpha]-synuclein aggregation", AUTOPHAGY, vol. 11, no. 10, 3 October 2015 (2015-10-03), pages 1745-1759, XP055664594, US ISSN: 1554-8627, DOI: 10.1080/15548627.2015.1082020
- B. MAIER ET AL: "A large-scale functional RNAi screen reveals a role for CK2 in the mammalian circadian clock", GENES AND DEVELOPMENT., vol. 23, no. 6, 15 March 2009 (2009-03-15) , pages 708-718, XP055664477, US ISSN: 0890-9369, DOI: 10.1101/gad.512209
- IANES, CHIARA et al.: "CKlð activity is modulated by CDK2/E- and CDK5/p35-mediated phosphorylating", Amino Acids, vol. 48, 31 December 2016 (2016-12-31), pages 579-592, XP035889336,
- TOH, KONG L. et al.: "An hPer2 Phosphorylation Site Mutation in Familial Advanced Sleep Phase Syndrome", SCIENCE, vol. 291, 9 February 2001 (2001-02-09), pages 1040-1043, XP055540585,

## Description

The present application claims the priority of the PCT patent application filed with China Intellectual Property Office, with the filing PCT/CN2017/075570, filed on March 3, 2017, entitled "Method and Compound for Modifying Circadian Clock".

### FIELD OF THE INVENTION

The present invention relates to a compound and composition modulating circadian rhythm as indicated in the appended claims.

### BACKGROUND OF THE INVENTION

With more than a billion cross-time zone travellers per year and 20% of Western workers taking shift-work (Roenneberg and Merrow, 2016), health problems associated with disturbances of the circadian rhythm are of considerable concerns (Ohlander et al., 2015; Parsons et al., 2015; Reutrakul and Knutson, 2015; Roenneberg et al., 2012; Schernhammer et al., 2003; Sigurdardottir et al., 2012; Tynes et al., 1996). However, the methods for treating circaidian related sleep problem and jet lag like melanopsin and GABA analogues target other molecules rather on the core clock of circadian rhythm. Therefore, compounds working on the core clock of circadian rhythm are desired. The circadian clock is an intrinsic timing system driving the daily rhythm of multiple systems including the sleep/wake cycle, immune responsiveness and metabolism (Asher and Sassone-Corsi, 2015; Bass and Takahashi, 2010; Chong et al., 2012; Curtis et al., 2014; Takahashi et al., 2008). In mammals, the master clock resides in the hypothalamic suprachiasmatic nucleus (SCN), which synchronizes circadian oscillations existing in cells throughout the body (Dibner et al., 2010; Mohawk and Takahashi, 2011; Welsh et al., 2010). Genetic studies in multiple organisms especially Drosophila and the mouse have established an interlocked transcription-translational feedback loop (TTFL) as the underlying molecular mechanisms of the circadian clock in animals (Allada et al., 2001; Andreani et al., 2015; Baker et al., 2012; Crane and Young, 2014; Hall, 2003; Hardin et al., 1990; Lowrey and Takahashi, 2011; Nitabach and Taghert, 2008; Panda et al., 2002; Reppert and Weaver, 2001; Zheng and Sehgal, 2012). In mammals, the first loop is the activation of three period genes (Per1, Per2, Per3) and two cryptochrome genes (Cry1, Cry2) by Bmal1 and Clock, while Per/Cry form a heterodimer to repress their own expression (Bunger et al., 2000; Gekakis et al., 1998; Griffin et al., 1999; Hogenesch et al., 1998; Kume et al., 1999; Reppert and Weaver, 2001; Shearman et al., 2000; Shearman et al., 1997; Shigeyoshi et al., 1997; Sun et al., 1997; Tei et al., 1997; van der Horst et al., 1999; Vitaterna et al., 1999; Zheng et al., 1999; Zylka et al., 1998). The second loop is the activation of two nuclear receptor genes (Rorα/β, Rev-erbα) by Bmal1 and Clock, while Rorα/β and Rev-erbα feedback on Bmal1 expression (Preitner et al., 2002; Sato et al., 2004; Ueda et al., 2002).

Posttranslational modifications, especially phosphorylation, are important in clock regulation (Crane and Young, 2014; Gallego and Virshup, 2007; Mehra et al., 2009; Reischl and Kramer, 2011). Both in Drosophila and in mammals, the PER protein(s), the prototypical circadian regulators, are phosphorylated and defective PER phosphorylation causes circadian disruption (Akashi et al., 2002; Blau, 2008; Chiu et al., 2011; Chiu et al., 2008; Cyran et al., 2005; Edery et al., 1994; Eide et al., 2005; Etchegaray et al., 2009; Gallego et al., 2006; Garbe et al., 2013; Iitaka et al., 2005; Kaasik et al., 2013; Kim et al., 2007; Kivimae et al., 2008; Kloss et al., 1998; Kloss et al., 2001; Ko et al., 2010; Lee et al., 2001; Lee et al., 2004; Lee et al., 2011; Lin et al., 2005; Maywood et al., 2014; Meng et al., 2008; Miyazaki et al., 2003; Miyazaki et al., 2004; Nawathean and Rosbash, 2004; Nawathean et al., 2007; Price et al., 1998; Reischl et al., 2007; Sathyanarayanan et al., 2004; Schmutz et al., 2011; Shanware et al., 2011; Shirogane et al., 2005; Takano et al., 2004; Toh et al., 2001; Tsuchiya et al., 2009b; Uchida et al., 2012; Vanselow et al., 2006; Vielhaber et al., 2000; Xu et al., 2005; Xu et al., 2007; Zhou et al., 2015). A point mutation resulting the switch from serine to glycine at the 662th amino acid residue in the human PER2 protein (hPER2) was discovered in a human family to correlate with the familial advanced sleep phase syndrome (FASPS) and proven to be causal in mice (Toh et al., 2001; Xu et al., 2007). hPER2 S662 was a priming site for casein kinase 1 (CK1 ), whose gene was found to be mutated in another family of FASPS(Xu et al., 2005; Xu et al., 2007). hPER2 has multiple potential phosphorylation sites, with CKs and glycogen synthetase kinase (GSK) 3 implicated in its phosphorylation (Akashi et al., 2002; Eide et al., 2005; Hirota et al., 2010; Iitaka et al., 2005; Lowrey et al., 2000; Maier et al., 2009; Meng et al., 2008; Tsuchiya et al., 2009a; Vanselow et al., 2006; Xu et al., 2005; Xu et al., 2007). It is likely that kinases other than CKs and GSK3 may also be involved in regulating the functions of hPER2 and other proteins involved in the circadian rhythm.

Chemical modifiers of circadian rhythm have been uncovered by investigations of inhibitors of CK1, GSK3 (Chen et al., 2013; Badura et al., 2007; Hirota et al., 2008; Isojima et al., 2009; Kennaway et al., 2015; Sprouse et al., 2010; Sprouse et al., 2009; Walton et al., 2009), REV-ERB (Solt et al., 2012) or vasopressin receptors (Yamaguchi et al., 2013). Screening of chemical libraries by cell-based circadian assays has also revealed chemical modifiers (Chen et al., 2012; Hirota and Kay, 2009; Hirota et al., 2010; Hirota et al., 2008; Isojima et al., 2009; Hirota et al., 2010; Hirota et al., 2012; Oshima et al., 2015). Keith Petrie ET AL: "Effect of melatonin on jet lag after long haul flights", 18 March 1989, pages 705-707 discloses the usefulness of melatonin in treating jet lag.

We initiated a strategy involving three steps to identify small molecule modifiers of the circadian rhythm: 1) we screened for kinases able to phosphorylate hPER2. The identification of new hPER2 kinases allowed us to narrow down the list of inhibitors as potential regulators of the clock; 2) we then used cultured human U2OS cells as a model to test inhibitors of hPER2 kinases for their ability to modify the human clock in vitro. We found several effective modifiers, of which we exemplify with dinaciclib in detail here. We demonstrate the effect of dinaciclib on brain slices containing the mammalian master clock and finally in intact animals. Dinaciclib significantly changed the phase of the clock not only in cultured U2OS cells but also in SCN slices. *in vivo* application of a single dose of dinaciclib to mice was effective in shortening the time required for adjustment in a 6 hour (h) advanced jet lag paradigm. We have therefore successfully used a rational approach to obtain compounds for modifying the phase of the circadian clock.

### SUMMARY OF THE INVENTION

In one aspect, the invention relates to dinaciclib for therapeutic use in the treatment of jet lag, shift-work or age-related sleep disturbances. In one embodiment, dinaciclib is in unit dosage form. In a further embodiment, dinaciclib is at a:
(a) dosage that is subtherapeutic for cancer treatment;
(b) dosage that is less than 50% , 20% or 10% of therapeutic or conventional cancer dosage;
(c) dosage that is less than 1 or 2 or 5 or 10 or 20 mg/m2 ; or
(d) unit dosage form of less than 1, 2, 5, 10 or 20 mg.

In another aspect, the invention relates to a composition characterized in comprising (a) dinaciclib, and (b) a different medicament for treating jet lag, shift-work or age-related sleep disturbances, selected from caffeine, melatonin, zolpidem, eszopiclone, zalepon or triazolam. In one embodiment, the composition comprises dinaciclib in unit dosage form. In a further embodiment, dinaciclib and the medicament are co-packaged or co-formulated. In a further embodiment, dinaciclib is at a:
(a) dosage that is subtherapeutic for cancer treatment;
(b) dosage that is less than 50%, 20% or 10% of therapeutic or conventional cancer dosage;
(c) dosage that is less than 1 or 2 or 5 or 10 or 20 mg/m2 ; or
(d) unit dosage form of less than 1, 2, 5, 10 or 20 mg.

In embodiments:
- dinaciclib is in unit dosage form, preferably enteral (e.g. oral);
- dinaciclib and the medicament are copackaged or coformulated, preferably in unit dosage form, preferably enteral;
- the subject is a person exposed or determined to be at risk of exposure to jet lag, shift-work or age-related sleep disturbances; and/or
- the use further comprises the antecedent step of determining the subject is exposed or at risk of exposure to jet lag, shift-work or age-related sleep disturbances.

### BRIEF DESCRIPTION OF THE FIGURES

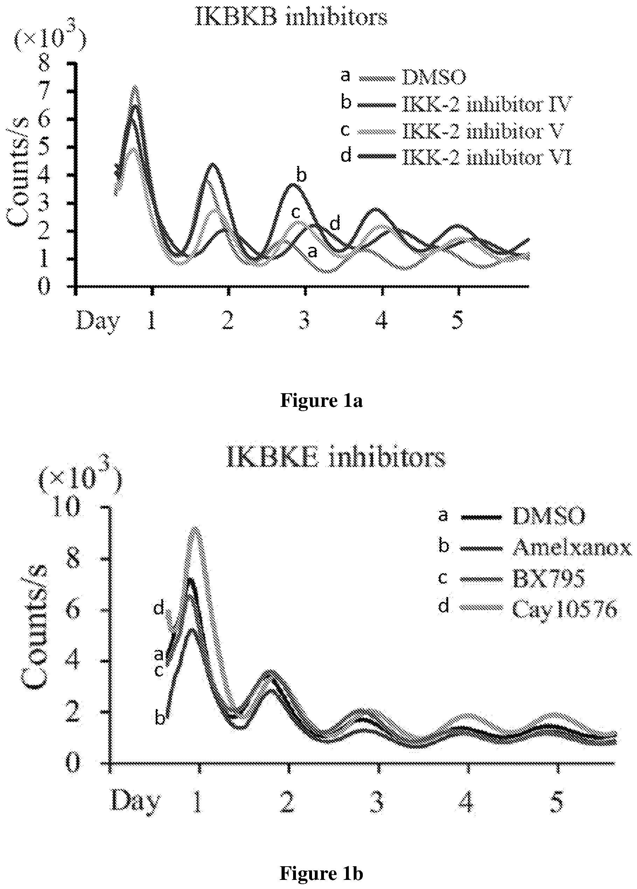

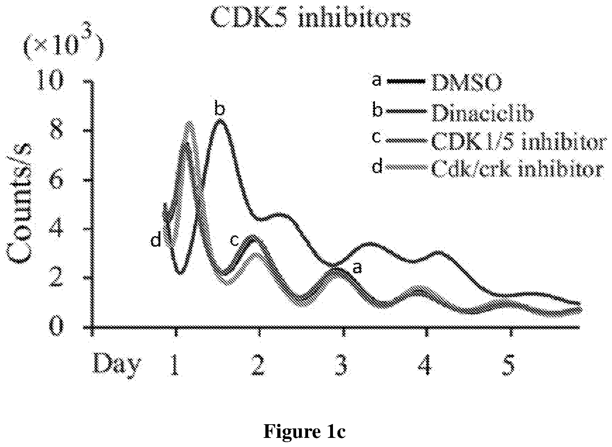

Figs. 1a, 1b and 1c: Effects of kinase inhibitors discovered in the chemical screen for their abilities to modify the circadian rhythm of U2OS cell. (a) IKBKB inhibitors. The concentration of all the inhibitors was 10µM, n=5. (b) IKBKE inhibitors. The concentration of the inhibitors was as follow: Amelxanox, 15 µM, n=4; Cay 10576, 15 µM, n=4; BX795, 0.3 µM, n=4 (c) CDK5 inhibitors. The concentration of the inhibitors was as follow: dinaciclib, 10 nM, n=4; Cdk/Crk inhibitor, 10nM, n=4; Cdk1/5 inhibitor, 10 µM, n=4. Dinaciclib caused the most dramatic shift in the phase of the circadian clock.

### DETAILED DESCRIPTION OF THE INVENTION

The detailed descriptions, particular embodiments and examples are provided by way of illustration and not by way of limitation.

Unless contraindicated or noted otherwise, in these descriptions and throughout this specification, the terms "a" and "an" mean one or more, the term "or" means and/or and polynucleotide sequences are understood to encompass opposite strands as well as alternative backbones described herein. Furthermore, genuses are recited as shorthand for a recitation of all members of the genus; for example, the recitation of (C1-C3) alkyl is shorthand for a recitation of all C1-C3 alkyls: methyl, ethyl and propyl, including isomers thereof.

### Dinaciclib formulation and administration

Dinaciclib, formulations and administration are readily empirically determined or otherwise known in the art, e.g. US7,119,200; US20160193334; WO 2015130585: Unless otherwise indicated, references below to dinaciclib also include pharmaceutically acceptable salts thereof. For preparing dinaciclib pharmaceutical compositions, inert, pharmaceutically acceptable carriers can be either solid or liquid. Solid form preparations include powders, tablets, dispersible granules, capsules, cachets and suppositories. The powders and tablets may be comprised of from about 5 to about 95 percent dinaciclib. Suitable solid carriers are known in the art, e.g., magnesium carbonate, magnesium stearate, talc, sugar or lactose. Tablets, powders, cachets and capsules can be used as solid dosage forms suitable for oral administration. Examples of pharmaceutically acceptable carriers and methods of manufacture for various compositions may be found in A. Gennaro (ed.), Remington 's Pharmaceutical Sciences, 18th Edition, (1990), Mack Publishing Co., Easton, Pennsylvania. Liquid form preparations of dinaciclib include solutions, suspensions and emulsions. As an example may be mentioned water or water-propylene glycol solutions for parenteral injection or addition of sweeteners and opacifiers for oral solutions, suspensions and emulsions. Liquid form preparations may also include solutions for intranasal administration. Aerosol preparations of dinaciclib suitable for inhalation may include solutions and solids in powder form, which may be in combination with a pharmaceutically acceptable carrier, such as an inert compressed gas, e.g. nitrogen. Also included are solid form preparations of dinaciclib that are intended to be converted, shortly before use, to liquid form preparations of dinaciclib for either oral or parenteral administration. Such liquid forms include solutions, suspensions and emulsions. Dinaciclib may also be deliverable transdermally. The transdermal compositions can take the form of creams, lotions, aerosols and/or emulsions and can be included in a transdermal patch of the matrix or reservoir type as are conventional in the art for this purpose. Dinaciclib may also be delivered subcutaneously. The pharmaceutical preparation can be in unit dosage form. In such form, the preparation is subdivided into suitably sized unit doses containing appropriate quantities of dinaciclib, e.g., an effective amount to achieve the desired purpose. The quantity of dinaciclib in a unit dose of preparation may be varied or adjusted from about 1 mg to about 100 mg, more specifically from about 1 mg to about 50 mg, more specifically from about 1 mg to about 25 mg, according to the particular application. The actual dosage employed may be varied depending upon the requirements of the patient and the severity of the condition being treated. Determination of the proper dosage regimen for a particular situation is within the skill of the art. For convenience, the total daily dosage may be divided and administered in portions during the day as required.

### Examples

Drugs regulating the circadian rhythm are desired to treat jet-lag, shift work-related problems and circadian-related sleep disorders in diseased or aging populations. Because the circadian clock involves protein phosphorylation, we first performed a screen for human kinases capable of phosphorylating fragments of the human Period 2 (hPER2) protein, leading to the findings of 6 kinases previously unknown for PER phosphorylation. We then performed a screen of kinase inhibitors for functional regulation of the circadian rhythm in cultured human cells. Several inhibitors were found to either lengthen or shorten the period, while one, dinaciclib, significantly shifted the phase of the circadian clock in a dose- and phase- dependent manner: up to 8.172±0.2194 hours (h) (mean±SEM, n=4 ). Dinaciclib could shift the phase of the superchiasmatic nucleus (SCN), the master clock in the mouse brain. A single injection of dinaciclib into mice significantly improved adjustment in a jet lag paradigm with 6h phase advancement. Thus, coupling chemical and biological screens effectively reveals small molecules of applications in clock shifting.

### A screen for kinases phosphorylating hPER2

To find kinases for hPER2, we subcloned and cotransfected cDNAs encoding kinases with different fragments of hPER2 in HEK293T cells. 288 protein kinases were successfully tested in our system. Phosphorylation of hPER2 was detected during the screen by the phos-tag which could bind to organic phosphate in proteins to delay their migration in sodium dodecyl sulfate-polyacrylaminde gel electrophoresis (SDS-PAGE) (Kinoshita et al., 2006). We divided the hPER2 with a molecular weight more than 130 kilodaltons (kD) into six fragments, each with a molecular weight between 20-30 kD. This was intended to increase the sensitivity of phosphorylation detection by gel-shift, and to separate the interference of phosphorylation on different sites. Because Fragments 771-1000 and 979-1240 showed multiple bands in HEK293T cells, our screen was focused on the remaining 4 fragments.

The screen uncovered zero kinase phosphorylating Fragment 1-200, one kinase phosphorylating Fragment 150-400, three kinases phosphorylating Fragment 328-556 and five kinases phosphorylating Fragment 556-771. Results from the screen were validated and that the gel shift was due to phosphorylation was confirmed by phosphatase treatment. One kinase phosphorylated both Fragment 328-556 and Fragment 556-771, while CSNK1D and CSNK1E have been previously identified (Akashi et al., 2002; Eide et al., 2005; Toh et al., 2001; Vanselow et al., 2006; Xu et al., 2005; Xu et al., 2007). This left us with 6 newly identified hPER2 phosphorylating kinases. Only one kinase, CDK5, was found to phosphorylate Fragment 150-400. PRKACB, PRKG1 and IKBKB can phosphorylate Fragment 328-556. PRKACB, TSSK2, IKBKE, CSNK1D and CSNK1E phosphorylate Fragment 556-771.

### Effects of kinase inhibitors on the circadian rhythm of cultured human cells (kinase inhibitors other than dinaciclib are not according to the invention)

We tested inhibitors of newly found kinases for their potential regulation of circadian rhythm in U2OS cells expressing *Per2*-luciferase which faithfully reported the circadian clock (Zhang et al., 2009). We did not detect any effect of PRKACB and PRKG1 inhibitors on circadian rhythm. Effects of CDK5, IKBKB and IKBKE inhibitors are shown in **Fig. 1** **and** Table 1.

**Table 1.**

| Kinase | Inhibitor | Phenotype |
|---|---|---|
| IKBKB | Ikk2 inhibitor iv | Period lengthening, 3.16±0.167h |
| | Ikk2 inhibitor v | Period lengthening, 1.84±0.192h |
| | Ikk2 inhibitor vi | Period lengthening, 1.25±0.149h |
| IKBKE | Amelxanox | N |
| | Cay10576 | Period shortening, 0.3±0.060h; phase delay |
| | Bx795 | N |
| CDK5 | Dinaciclib | Period shortening, 0.6±0.050h; phase delay |
| | Cdk/crk inhibitor | Period shortening, 0.5±0.074h; phase delay |
| | Cdk1/5 inhibitor | N |

All IKBKB inhibitors tested here lengthened the period. Three inhibitors of IKBKE showed different effects: Amelxanox and BX795 had no detectable effect whereas Cay10576 shortened the period by 0.3 h. Three inhibitors of CDK5 also showed different effects. Interestingly, dinaciclib significantly shifted the phase of the circadian rhythm of U2OS cells.

### Effects of dinaciclib on the circadian rhythm of human cells

To further test the effect of dinaciclib, we used a cell line expressing *Bmal1*-luciferase, which oscillated in a phase different from *Per*2-luciferase. The phase-shifting effect of dinaciclib was confirmed in *Bmal1*-luciferase cells, supporting that dinaciclib affected the circadian rhythm, rather than just the expression of one reporter gene specifically. When the timing of first peak of *Per2*-luciferase expression was used to calculate the extent of phase shift, dinaciclib was found to delay the peak by 9.408h. When applied from the beginning of the assay, dinaciclib increased the amplitude by 1.78 fold and shortened the period by 0.6 h.

We examined the dosage response of dinaciclib on *Per2*-luciferase expressing cells. The effects of dinaciclib on phase shifting, amplitude and period length were all dosage dependent. The EC₅₀ (the half maximal effective concentration) of dinaciclib for phase shifting was 8.363nM.

The above experiments were conducted with the drug added to the culture media from the beginning. To investigate whether the timing of drug treatment affected the response, dinaciclib was added to *Per2*-luciferase expressing cells at different circadian time (CT). Dinaciclib delayed the phase when applied at CT0 by 4.092±0.9062h (p<0.01, n=5), at CT6 by 7.543±0.6185h (p<0.001, n=5) and at CT12 by 5.808±0.5455h (p<0.0001, n=5). However, the circadian phase was advanced by 1.7±0.81h if dinaciclib was introduced at CT18. In summary, dinaciclib modification of the circadian rhythm is both dose- and phase-dependent.

### Effect of dinaciclib on the circadian rhythm in the SCN

U2OS cells are just cultured cells, while the SCN is the master clock in mammals. We dissected the SCN from mice expressing the Per2::Luciferase gene which faithfully reported the circadian rhythm (Yoo et al., 2004). SCN explants were cut into slices which maintained rhythmicity not only cell-autonomously but also in a cell-cell interaction dependent manner. Application of dinaciclib at CT12 advanced the phase of SCN explants by 2.488±0.7391h (p<0.01, n=8), whereas dinaciclib application at CT0 or CT18 delayed the phase. Differences between SCN slices and isolated tissue culture cells may result from interaction among SCN cells required for synchronization.

Thus, dinaciclib can regulate the circadian rhythm in SCN slices containing the mammalian master clock. It significantly advanced the SCN phase when applied at CT12:00.

### In vivo effect of dinaciclib on a jet lag paradigm of mice

That dinaciclib can modify the phase of the circadian rhythm in cultured human cells and mouse SCN explants suggests the exciting possibility that dinaciclib can be a powerful drug to treat jet lag in intact animals.

After adult mice were placed in a light/dark (LD) cycle of 12L:12D (light on at CT 3:00, light off at CT15:00) for 11 days, dinaciclib (0.1 mg/kg body weight) was applied at CT20:00. At CT21:00, the LD cycle was advanced by 6 h (light on at CT21:00, light off at CT9:00), and the mice were kept in the new LD for 14 days.

Compared to the vehicle treated mice, mice treated with a single dose of dinaciclib adapted to the new LD cycle in a significantly shorter time. The 50% phase shift time (PS₅₀) for the dinaciclib-treated group was 1.34 days while that for the control was 2.63 days (*p*<0.01). Thus, dinaciclib was effective in treating jet lag.

Our screen for kinases phosphorylating hPER2, a clock component, facilitated the screen for small molecules regulating the clock. The discovery of dinaciclib from the screens was extended by further analysis of its effects in cultured human cells, brain slices and intact mice.

Dinaciclib is an anti-cancer drug with no previous evidence to indicate that it could affect the circadian rhythm (Parry et al., 2010; Paruch et al., 2010). The facts that it has passed phase I and phase II of clinical trials and that the dose used for jet lag here is lower than that for cancer treatment support the possibility that dinaciclib is relatively safe (Fabre et al., 2014; Flynn et al., 2015; Gojo et al., 2013; Gorlick et al., 2012; Hu et al., 2015; Kumar et al., 2015; Mita et al., 2014; Mitri et al., 2015; Nemunaitis et al., 2013a; Stephenson et al., 2014; Zhang et al., 2012).

By screening 288 protein kinases, we have found 6 new kinases capable of phosphorylating hPER2. We tested typical inhibitors for their ability to modify the circadian rhythm in cultured human U2OS cells. CDK5 could phosphorylate hPER2 Fragment 150-400. CDK5 is an atypical cyclin dependent kinase which is activated by P35 or P39 instead of cyclin (Dhavan and Tsai, 2001). Fragment 150-400 contains one PAS domain, which is for the transcriptional regulatory activity of PER proteins. However, there was a report that CDK5 could phosphorylate the CLOCK protein (Kwak et al., 2013).

The immune system is under the control of circadian rhythm (Curtis et al., 2014; Scheiermann et al., 2013). In our kinase screen, IKBKB and IKBKE, two kinases known to be important in the immune system (Chen, 2012), could phosphorylate hPER2 fragments. Here we have also found that IKBKB inhibitors could lengthen the period of U2OS. The degradation of hPER2 requires the ubiquitination in the region 328-771 which is also phosphorylation dependent (Eide et al., 2005; Ohsaki et al., 2008). For example, the S662G mutation found in FASPS is in this region (Toh et al., 2001).

Dinaciclib is currently being investigated in clinical trials for a number of tumors, including refractory chronic lymphocytic leukemia (phase III). It potently and selectively inhibits CDK5, CDK9, CDK1 and CDK2 (Fabre et al., 2014; Flynn et al., 2015; Gojo et al., 2013; Gorlick et al., 2012; Guzi et al., 2011; Hu et al., 2015; Kumar et al., 2015; Mita et al., 2014; Mitri et al., 2015; Nemunaitis et al., 2013a; Nemunaitis et al., 2013b; Parry et al., 2010; Paruch et al., 2010; Stephenson et al., 2014; Zhang et al., 2012). Among the CDK inhibitors evaluated, dinaciclib is the most potent and has a unique kinase selectivity profile, though it remains to be investigated which CDK is the endogenous target of dinaciclib for its modification of the circadian rhythm. Our work indicates that regulators of kinase activities can provide effective and safe modifiers of the circadian rhythm, alleviating problems associated with not only jet lag, but also shift-work and age related sleep disturbances.

### Mouse jet lag assay

The tip of the cannula was inserted stereotaxically to the lateral ventricular of mice. After surgery, mice were returned to the home cage under a LD cycle (light on at CT3:00, light off at CT15:00) for 7 days before video recording of mouse activities began. After another four days, at CT 20:00, Dinaciclib dissolved in 20% hydroxypropyl-β-cyclodextran (Parry et al., 2010) (at the concentration of 5mg/ml, at the speed of 0.5 1/min) or vehicle alone was pumped into the brain to achieve the total amount of 0.1 mg/kg of body weight by the UltraMicroPump III under the help of Micro4 Controller. At CT21:00 of the same day, the LD cycle was shifted to light on at CT21:00, light off at CT9:00, which was 6hrs advanced. Mice were kept in the new LD for 14 days. Data was processed and moving distance/30s was calculated.

### Kinases

CDK5 (cyclin-dependent kinase 5); PRKACB (protein kinase cAMP-activated catalytic subunit beta); PRKG1 (Protein kinase cGMP-dependent, Type 1); IKBKB (also IKK2) - Inhibitor of nuclear factor kappa-B kinase subunit beta; IKBKE (Inhibitor of nuclear factor kappa-B kinase subunit epsilon); TSSK2 (testis specific serine kinase 2).

### Kinase inhibitors (kinase inhibitors other than dinaciclib are not according to the invention)

Dinaciclib, (2-[(2S)-1-[3-ethyl-7-[(1-oxidopyridin-1-ium-3-yl)methylamino]pyrazolo[1,5-a]pyrimidin-5-yl]piperidin-2-yl]ethanol), Amelxanox: (2-amino-5-oxo-7-propan-2-ylchromeno[2,3-b]pyridine-3-carboxylic acid), Cdk1/5 inhibitor (3-Amino-1H-pyrazolo[3,4-b]quinoxaline), Cdk/Crk inhibitor: (1-(2,6-dichlorophenyl)-6-[[4-(2-hydroxyethoxy)phenyl]methyl]-3-propan-2-yl-2H-pyrazolo[ 3,4-d]pyrimidin-4-one), IKK-2 inhibitor IV (TPCA-1, or 2-(carbamoylamino)-5-(4-fluorophenyl)thiophene-3-carboxamide), IKK-2 inhibitor V (IMD 0534, or N-[3,5-bis(trifluoromethyl)phenyl]-5-chloro-2-hydroxybenzamide), IKK-2 inhibitor VI (2-[(aminocarbonyl)amino]-5-phenyl-3-thiophenecarboxamide)), Cay10576 (5-(5,6-dimethoxybenzimidazol-1-yl)-3-[(2-methylsulfonylphenyl)methoxy]thiophene-2-carb onitrile), BX795 (N-[3-[[5-iodo-4-[3-(thiophene-2-carbonylamino)propylamino]pyrimidin-2-yl]amino]phenyl] pyrrolidine-1-carboxamide), KT5720 (Hexyl 6-hydroxy-5-methyl-13-oxo-5,6,7,8,14,15-hexahydro-13h-5,8-epoxy-4b,8a,14-triazadibenzo[ b,h]cycloocta[1,2,3,4-jkl]cyclopenta[e]-as-indacene-6-carboxylate), Rp-cAMPS ((4aR,6R,7R,7aS)-6-(6-aminopurin-9-yl)-2-oxido-2-sulfanylidene-4a,6,7,7a-tetrahydro-4H-fu ro[3,2-d][1,3,2]dioxaphosphinin-7-ol;triethylazanium), KT5823 (9-methoxy-9-methoxycarbonyl-8-methyl-2,3,9,10-tetrahydro-8,11-epxoy-1H,8H,11H-2,7b-1 1a-triazadibenzo(a,g)cycloocta(cde)-trinden-1-one), Rp-8-Br-PET-cGMPS (sodium;3-[(4aR,6R,7R,7aS)-7-hydroxy-2-oxido-2-sulfanylidene-4a,6,7,7a-tetrahydro-4H-fur o[3,2-d][1,3,2]dioxaphosphinin-6-yl]-2-bromo-6-phenyl-5H-imidazo[1,2-a]purin-9-one).

### References

Akashi, M., et al. (2002). Mol and Cellular Biol 22, 1693-1703.
Allada, R., et al. (2001). S. Annu Rev Neurosci 24, 1091-1119.
Andreani, T.S., et al. (2015). Sleep medicine clinics 10, 413-421.
Asher, G, and Sassone-Corsi, P. (2015).. Cell 161, 84-92.
Badura, L., et al. (2007). J Pharmacol and Exper Therapeutics 322, 730-738.
Baker, C.L., et al. (2012). FEMS microbiology reviews 36, 95-110.
Bass, J., and Takahashi, J.S. (2010). Science 330, 1349-1354.
Blau, J. (2008). Genes & development 22, 1737-1740.
Bunger, M.K., et al. (2000). Cell 103, 1009-1017.
Cao, R., et al. (2013). Neuron 79, 712-724.
Chen, Z., et al.PNAS USA 109, 101-106.
Chen, Z., et al. (2013). Cellular and mol life sciences 70, 2985-2998.
Chen, Z.J. (2012). Immunological reviews 246, 95-106.
Chiu, J.C., Ko, H.W., and Edery, I. (2011). Cell 145, 357-370.
Chiu, J.C., et al. (2008). Genes & development 22, 1758-1772.
Chong, S.Y, et al. (2012). Trends in genetics 28, 598-605.
Crane, B.R., and Young, M.W. (2014). Annual review of biochem 83, 191-219.
Curtis, A.M., et al. (2014). Immunity 40, 178-186.
Cyran, S.A., et al. (2005). J of Neurosci 25, 5430-5437.
Dhavan, R., and Tsai, L.H. (2001). Nat Rev Mol Cell Bio 2, 749-759.
Dibner, C., et al. (2010). Annual review of physiology 72, 517-549.
Edery, I., et al. (1994). PNAS USA 91, 2260-2264.
Eide, E.J., et al. (2005). Molecular and cellular biology 25, 2795-2807.
Etchegaray, J.P, et al. (2009). Mol cellular biolo 29, 3853-3866.
Fabre, C., et al. (2014). Cancer chemother and pharmacol 74, 1057-1064.
Flynn, J., et al. (2015). Leukemia 29, 1524-1529.
Gallego, M., et al. (2006). The Biochemical J 399, 169-175.
Gallego, M., and Virshup, D.M. (2007). Nat Rev Mol Cell Bio 8, 139-148.
Garbe, D.S., et al. (2013). Plos Genet 9, e1003749.
Gekakis, N., et al. (1998). Science 280, 1564-1569.
Gojo, I., Sadowska, M., et al. (2013). Cancer chemother and pharmacol 72, 897-908.
Gorlick, R., et al. (2012). Pediatr Blood Cancer 59, 1266-1274.
Griffin, E.A., et al. (1999). Science 286, 768-771.
Guzi, T.J., et al. (2011). Mol Cancer Ther 10, 591-602.
Hall, J.C. (2003). Adv Genet 48, 1-280.
Hardin, P.E., Hall, J.C., and Rosbash, M. (1990). Nature 343, 536-540.
Hirota, T., and Kay, S.A. (2009). Chemistry & biology 16, 921-927.
Hirota, T., et al. (2010). PLoS biology 8, e1000559.
Hirota, T., et al. (2012). Science 337, 1094-1097.
Hirota, T., et al. (2008). PNAS USA 105, 20746-20751.
Hogenesch, J.B., et al. (1998). PNAS USA 95, 5474-5479.
Hu, C., et al. (2015). Mol Cancer Ther 14, 1532-1539.
Iitaka, C., et al. (2005). J Biol Chem 280, 29397-29402.
Isojima, Y, et al. (2009). PNAS USA 106, 15744-15749.
Johannessen, et al. (2010). Nature 468, 968-972.
Kaasik, et al. (2013). Cell Metab 17, 291-302.
Kennaway, D.J., et al.. (2015). Mol and cellular biochem 398, 195-206.
Kim, E.Y, et al. (2007). Mol and cellular biol 27, 5014-5028.
Kinoshita, E., et al. (2006). Mol Cell Proteomics 5, 749-757.
Kivimae, S., et al. (2008). PLoS biology 6, e183.
Kloss, B., et al. (1998). Cell 94, 97-107.
Kloss, B., et al. (2001). Neuron 30, 699-706.
Ko, H.W., et al. (2010). J of Neurosci 30, 12664-12675.
Kumar, S.K., et al. (2015). Blood 125, 443-448.
Kume, K., et al. (1999). Cell 98, 193-205.
Kwak, Y, et al. (2013). J Biol Chemi 288, 36878-36889.
Langmesser, S., et al. (2009). PloS one 4*.*
Lee, C., et al. (2001). Cell 107, 855-867.
Lee, C., et al. (2004). Mol and cellular biol 24, 584-594.
Lee, H.M., et al. (2011). PNAS USA 108, 16451-16456.
Levine, J.D., et al. (1994). Neuron 13, 967-974.
Li, Y, et al. (2011).Mol human reproduction 17, 42-56.
Lin, J.M., et al. (2005). J Neurosci 25, 11175-11183.
Lowrey, P.L., et al. (2000). Science 288, 483-492.
Lowrey, P.L., et al. (2011). Genetics Of Circadian Rhythms 74, 175-230.
Maier, B., et al. (2009). Genes & dev 23, 708-718.
Maywood, E.S., et al. (2014). J of biological rhythms 29, 110-118.
Mehra, A., et al. (2009). Trends in biochem sciences 34, 483-490.
Meng, Q.J., et al. (2008). Neuron 58, 78-88.
Mita, M.M., et al. (2014). Clinical breast cancer 14, 169-176.
Mitri, Z., et al. (2015). Investigational new drugs 33, 890-894.
Miyazaki, K., et al. (2003). Novartis Found symposium 253, 238-248; discussion 249.
Miyazaki, K., et al. (2004). The Biochemical journal 380, 95-103.
Mohawk, J.A., and Takahashi, J.S. (2011). Trends in neurosciences 34, 349-358.
Nawathean, P., and Rosbash, M. (2004). Mol Cell 13, 213-223.
Nawathean, P., et al. (2007). Mol and cellular biol 27, 5002-5013.
Nemunaitis, J.J., et al. (2013a). J Transl Med 11, 259.
Nemunaitis, J.J., et al. (2013b). J Transl Med 11*.*
Nijhawan, D., et al. (2003). Genes & development 17, 1475-1486.
Nitabach, M.N. and Taghert, P.H. (2008). Current biol 18, R84-93.
Ohlander, J., et al. (2015). Am J industrial med 58, 549-560.
Ohsaki, K., et al. (2008). Journal of biochemistry 144, 609-618.
Oshima, T., et al. (2015). Angew Chem Int Edit 54, 7193-7197.
Panda, S., et al. (2002). Nature 417, 329-335.
Parry, D., et al. (2010). Mol Cancer Ther 9, 2344-2353.
Parsons, M.J., et al. (2015). Int J Obes (Lond) 39, 842-848.
Paruch, K., et al. (2010). ACS medicinal chemistry letters 1, 204-208.
Preitner, N., et al. (2002). Cell 110, 251-260.
Price, J.L., et al. (1998). Cell 94, 83-95.
Reed, S.E., et al. (2006). J Virol Methods 138, 85-98.
Reischl, S., and Kramer, A. (2011). FEBS letters 585, 1393-1399.
Reischl, S., et al. (2007). J biological rhythms 22, 375-386.
Reppert, S.M., and Weaver, D.R. (2001). Annual review of physiology 63, 647-676.
Reutrakul, S., and Knutson, K.L. (2015). Sleep medicine clinics 10, 455-468.
Roenneberg, T., et al. (2012). Current biology : CB 22, 939-943.
Sathyanarayanan, S., et al. (2004). Cell 116, 603-615.
Sato, T.K., et al.Neuron 43, 527-537.
Savelyev, S.A., et al. (2011). Journal of visualized experiments
Scheiermann, C., et al. (2013). Nature reviews Immunology 13, 190-198.
Schernhammer, E.S., et al. (2003). J Natl Cancer Inst 95, 825-828.
Schmutz, I., et al. (2011). PloS one 6, e21325.
Shanware, N.P., et al. (2011). J biol chem 286, 12766-12774.
Shearman, L.P., et al. (2000). Science 288, 1013-1019.
Shearman, L.P., et al. (1997). Neuron 19, 1261-1269.
Shigeyoshi, Y, et al. (1997).. Cell 91, 1043-1053.
Shirogane, T., et al. (2005). J biological chemistry 280, 26863-26872.
Sigurdardottir, L.G, et al. (2012). Cancer epidemiol, biomarkers & preven 21, 1002-1011.
Solt, L.A., et al. (2012). Nature 485, 62-68.
Sprouse, J., et al. (2010). Psychopharmacology 210, 569-576.
Sprouse, J., et al. (2009). Psychopharmacology 204, 735-742.
Stephenson, J.J., et al. (2014). Lung cancer 83, 219-223.
Sun, Z.S., et al. (1997). Cell 90, 1003-1011.
Takahashi, J.S., et al. (2008). Nature reviews Genetics 9, 764-775.
Takano, A., et al. (2004). J Biol chem 279, 32578-32585.
Tei, H., et al. (1997). Nature 389, 512-516.
Toh, K.L., et al. (2001). Science 291, 1040-1043.
Tsuchiya, Y, et al. (2009a).. Science signaling 2*.*
Tsuchiya, Y, et al. (2009b). Science signaling 2, ra26.
Tynes, T., et al.1996).. Cancer causes & control 7, 197-204.
Uchida, Y, et al. (2012).. J Biol chemistry 287, 8318-8326.
Ueda, H.R., et al. (2002). Nature 418, 534-539.
van der Horst, G.T.J., et al. (1999). Nature 398, 627-630.
Vanselow, K., et al. (2006). Genes & development 20, 2660-2672.
Vielhaber, E., et al.2000). Mol and cellular biol 20, 4888-4899.
Vitaterna, M.H., et al. (1999). PNAS USA 96, 12114-12119.
Walton, K.M., et al. (2009). J pharmacol and exp therapeutics 330, 430-439.
Welsh, D.K., et al. (2010). Annual review of physiology 72, 551-577.
Xu, Y, et al. (2005). Nature 434, 640-644.
Xu, Y, et al. (2007). Cell 128, 59-70.
Yamaguchi, Y, et al. (2013). Science 342, 85-90.
Yoo, S.H., et al. (2004). PNAS USA 101, 5339-5346.
Zhang, D., et al. (2012). Cancer chemotherapy and pharmacol 70, 891-898.
Zheng, B., et al. (1999). Nature 400, 169-173.
Zheng, X., and Sehgal, A. (2012). Trends in neurosciences 35, 574-585.
Zhou, M., et al. (2015). Mol Cell 60, 77-88.
Zylka, M.J., et al. (1998). Neuron 20, 1103-1110.

## Claims

1. Dinaciclib for therapeutic use in the treatment of jet lag, shift-work or age-related sleep disturbances.

2. Dinaciclib for therapeutic use according to claim 1, wherein the dinaciclib is in unit dosage form

3. Dinaciclib for therapeutic use according to claim 1 or 2, wherein the dinaciclib is at a
(a) dosage that is subtherapeutic for cancer treatment;
(b) dosage that is less than 50% , 20% or 10% of therapeutic or conventional cancer dosage;
(c) dosage that is less than 1 or 2 or 5 or 10 or 20 mg/m²; or
(d) unit dosage form of less than 1, 2, 5, 10 or 20 mg.

4. A composition **characterized in** comprising (a) dinaciclib, and (b) a different medicament for treating jet lag, shift-work or age-related sleep disturbances, selected from caffeine, melatonin, zolpidem, eszopiclone, zalepon or triazolam.

5. The composition according to claim 4, wherein the dinaciclib is in unit dosage form.

6. The composition according to claim 4 or 5, wherein the dinaciclib and medicament are co-packaged or co-formulated.

7. The composition according to any of the claims 4 to 6, wherein the dinaciclib is at a:
(a) dosage that is subtherapeutic for cancer treatment;
(b) dosage that is less than 50%, 20% or 10% of therapeutic or conventional cancer dosage;
(c) dosage that is less than 1 or 2 or 5 or 10 or 20 mg/m²; or
(d) unit dosage form of less than 1, 2, 5, 10 or 20 mg.

## Patentansprüche

1. Dinaciclib zur therapeutischen Verwendung bei der Behandlung von Jetlag, Schichtarbeit oder altersbedingten Schlafstörungen.

2. Dinaciclib zur therapeutischen Verwendung nach Anspruch 1, wobei das Dinaciclib in Einheitsdosierungsform vorliegt.

3. Dinaciclib zur therapeutischen Verwendung nach Anspruch 1 oder 2, wobei das Dinaciclib vorliegt als:
(a) Dosierung, die für die Krebsbehandlung subtherapeutisch ist;
(b) eine Dosis, die weniger als 50 %, 20 % oder 10 % der therapeutischen oder herkömmlichen Krebsdosis beträgt;
(c) eine Dosis, die weniger als 1 oder 2 oder 5 oder 10 oder 20 mg/m² beträgt, oder
(d) eine Einzeldarreichungsform von weniger als 1, 2, 5, 10 oder 20 mg.

4. Zusammensetzung, **dadurch gekennzeichnet, dass** sie (a) Dinaciclib und (b) ein anderes Medikament zur Behandlung von Jetlag, Schichtarbeit oder altersbedingten Schlafstörungen, ausgewählt aus Koffein, Melatonin, Zolpidem, Eszopiclon, Zalepon oder Triazolam, umfasst.

5. Zusammensetzung nach Anspruch 4, wobei das Dinaciclib in Einheitsdosierungsform vorliegt.

6. Zusammensetzung nach Anspruch 4 oder 5, wobei das Dinaciclib und das Arzneimittel gemeinsam verpackt oder zusammen formuliert sind.

7. Zusammensetzung nach einem der Ansprüche 4 bis 6, wobei das Dinaciclib vorliegt als:
(a) Dosierung, die für die Krebsbehandlung subtherapeutisch ist;
(b) eine Dosis, die weniger als 50 %, 20 % oder 10 % der therapeutischen oder herkömmlichen Krebsdosis beträgt;
(c) eine Dosis, die weniger als 1 oder 2 oder 5 oder 10 oder 20 mg/m² beträgt, oder
(d) eine Einzeldarreichungsform von weniger als 1, 2, 5, 10 oder 20 mg.

## Revendications

1. Dinaciclib à usage thérapeutique pour le traitement des troubles du sommeil liés au décalage horaire, au travail posté ou à l'âge.

2. Dinaciclib à usage thérapeutique selon la revendication 1, dans lequel le dinaciclib est sous forme de dose unitaire.

3. Dinaciclib à usage thérapeutique selon la revendication 1 ou 2, dans lequel le dinaciclib est à :
(a) une dose sous-thérapeutique pour le traitement du cancer ;
(b) une dose qui est inférieure à 50 %, 20 % ou 10 % de la dose thérapeutique ou habituelle pour un cancer ;
(c) une dose inférieure à 1 ou 2 ou 5 ou 10 ou 20 mg/m² ; ou
(d) une forme de dose unitaire inférieure à 1, 2, 5, 10 ou 20 mg.

4. Composition **caractérisée en ce qu'**elle comprend (a) du dinaciclib, et (b) un médicament différent pour traiter les troubles du sommeil liés au décalage horaire, au travail posté ou à l'âge, choisi parmi la caféine, la mélatonine, le zolpidem, l'eszopiclone, le zaléplone ou le triazolam.

5. Composition selon la revendication 4, dans laquelle le dinaciclib est sous une forme de dose unitaire.

6. Composition selon la revendication 4 ou 5, dans laquelle le dinaciclib et le médicament sont co-conditionnés ou co-formulés.

7. Composition selon l'une quelconque des revendications 4 à 6, dans laquelle le dinaciclib est à :
(a) une dose sous-thérapeutique pour le traitement du cancer ;
(b) une dose qui est inférieure à 50 %, 20 % ou 10 % de la dose thérapeutique ou habituelle pour un cancer ;
(c) une dose inférieure à 1 ou 2 ou 5 ou 10 ou 20 mg/m² ; ou
(d) une forme de dose unitaire inférieure à 1, 2, 5, 10 ou 20 mg.
